# EUROPEAN PATENT APPLICATION

(11) **EP 3 482 636 A1**
(43) Date of publication of application: **15.05.2019**
(21) Application number: 18205058.3
(22) Date of filing: 08.11.2018
(51) Int. Cl.: A23C 9/123, C12R 1/46, A23L 33/135

(54) **PREPARATION OF FERMENTED DAIRY PRODUCTS FOR AMBIENT DISTRIBUTION**

(30) Priority: 10.11.2017 EP 17201050
(71) Applicant: Tetra Laval Holdings & Finance S.A., 1009 Pully (CH)
(72) Inventor: Lindgren, Katarina, 22733 Lund (SE); Cronström, Carin, 26332 Höganäs (SE); Björklund, Dan, 224 67 Lund (SE)
(74) Representative: Tetra Pak - Patent Attorneys SE

(57) **Abstract**

A method is provided for producing packages containing a fermented dairy product for ambient distribution. The method comprises providing (101) a dairy product precursor, processing (102) the dairy product precursor to generate the fermented dairy product via an intermediate product, and dispensing (103) the fermented dairy product into the packages for ambient distribution. The processing (102) comprises: performing, on the dairy product precursor or the intermediate product, a final treatment (102A) for destruction of microorganisms; and adding (102B) to the dairy product precursor or the intermediate product, subsequent to the final treatment (102A), a dedicated culture which is free of bacteria that consume lactose and which comprises lactic acid bacteria that consume at least one type of sugar other than lactose, so that the fermented dairy product in the packages comprises a live bacterial culture originating from the dedicated culture. The method may be implemented by an arrangement comprising a sequence of suitably configured stations.

## Description

### Technical Field

The invention generally relates to preparation of dairy products, and in particular to preparation of fermented dairy products for ambient distribution and storage.

### Background Art

In many countries, infrastructure is inadequate or completely missing for distributing food at chilled conditions, e.g. at a maximum temperature of 8-10°C. To make safe food available everywhere, food products aimed for distribution at ambient temperatures, e.g. 15-40°C, has become increasingly important. Food products for ambient distribution, also known as shelf-stable food products, can also provide advantages to consumers even if chilled food products are available, e.g. the convenience of bringing a meal or snack on the go, obviating the need for refrigerators, etc.

Fermented dairy products are prepared by adding a fermentation culture containing lactic acid bacteria to a starting material, e.g. a mixture of milk and any further ingredients such as sugar, stabilizer, etc. The lactic acid bacteria consume the sugar in the milk (lactose) and produces lactic acid and aroma. The increased amount of lactic acid lowers the pH of the mixture, causing an aggregation of proteins. When enough lactic acid and aroma have been produced, the mixture is cooled down in order to slow down the activity of the lactic acid bacteria. For some types of fermented dairy products, the fermented mixture is then further processed, e.g. concentrated to increase protein and fat content or homogenized to make the product more liquid.

Generally, fermented dairy products require chilled storage and distribution to limit the activity of fermentation culture and/or any spoilage of microorganisms that may be present in the products, e.g. due to recontamination. In order to create a product that can be stored and distributed at ambient conditions, a final treatment of the fermented mixture is required to inactivate or destruct the fermentation culture and spoilage microorganisms, if present. The final treatment conventionally involves a heat treatment, which typically affects the fermented mixture in other ways than by destructing microorganisms, for instance by lowering its viscosity and stability. Such effects may be at least partly counteracted by addition of stabilizing and texturizing ingredients. The heat treatment may also have an undesired impact on the flavor of the fermented mixture.

Many countries impose regulations on certain types of fermented dairy products, e.g. by requiring presence of live bacteria. For example, regulations may stipulate that to be named "yoghurt", a fermented product must contain a certain amount of live bacteria. Live fermentation bacteria is also perceived to result in health benefits. As understood from the foregoing, currently available fermented dairy products for ambient distribution and sales to consumers are free of live bacteria.

### Summary

It is an object of the invention to at least partly overcome one or more limitations of the prior art.

One such object is to provide a fermented dairy product with live bacteria for ambient distribution.

Another object is to reduce perceived differences, e.g. in taste and/or texture, between fermented dairy products for ambient distribution and for chilled distribution.

One or more of these objects, as well as further objects that may appear from the description below, are at least partly achieved by a method, a package and an arrangement according to the independent claims, embodiments thereof being defined by the dependent claims.

A first aspect of the invention is a method of producing packages containing a fermented dairy product for ambient distribution. The method comprises: providing a dairy product precursor; processing the dairy product precursor to generate the fermented dairy product via an intermediate product; and dispensing the fermented dairy product into the packages for ambient distribution. The processing comprises: performing, on the dairy product precursor or the intermediate product, a final treatment for destruction of microorganisms; and adding to the dairy product precursor or the intermediate product, subsequent to the final treatment, a dedicated culture which is free of bacteria that consume lactose and which comprises lactic acid bacteria that consume at least one type of sugar other than lactose, so that the fermented dairy product in the packages comprises a live bacterial culture originating from the dedicated culture.

A second aspect of the invention is a package produced by the method of the first aspect.

A third aspect of the invention is an arrangement for producing packages containing a fermented dairy product for ambient distribution. The arrangement comprises: a preparatory station configured to provide a dairy product precursor; a sequence of processing stations configured to process the dairy product precursor into the fermented dairy product via an intermediate product; and a packaging station configured to receive the fermented dairy product from the sequence of processing stations and dispense the fermented dairy product into the packages for ambient distribution. The sequence of processing stations comprises: a first station which is configured to perform, on the dairy product precursor or the intermediate product, a final treatment for destruction of microorganisms; and a second station which is arranged downstream of the first station and configured to add, to the dairy product precursor or the intermediate product, a dedicated culture which is free of bacteria that consume lactose and which comprises lactic acid bacteria that consume at least one type of sugar other than lactose, so that the fermented dairy product comprises a live bacterial culture originating from the dedicated culture.

Still other objectives features, aspects and advantages of the invention will appear from the following detailed description as well as from the drawings.

### Brief Description of the Drawings

Embodiments of the invention will now be described, by way of example, with reference to the accompanying schematic drawings.
FIG. 1 is a flow chart of a production process in accordance with an embodiment.
FIGS 2-3 are flow charts of first and second implementation concepts of the embodiment in FIG. 1.
FIG. 4 is a functional block diagram of an arrangement for producing packages containing a fermented dairy product for ambient distribution in accordance with an embodiment.

### Detailed Description

Embodiments of the present invention will now be described more fully hereinafter with reference to the accompanying drawings, in which some, but not all, embodiments of the invention are shown. Indeed, the invention may be embodied in many different forms and should not be construed as limited to the embodiments set forth herein; rather, these embodiments are provided so that this disclosure may satisfy applicable legal requirements.

Well-known functions or constructions may not be described in detail for brevity and/or clarity. Unless otherwise defined, all terms (including technical and scientific terms) used herein have the same meaning as commonly understood by one of ordinary skill in the art to which this invention belongs.

Like reference signs refer to like elements throughout.

Before describing embodiments of the invention in more detail, a few definitions will be given.

As used herein, "fermented dairy product" refers to a dairy food product that has been prepared by fermentation of a substance that contains milk from mammals, primarily but not exclusively cattle, or milk from plant sources. The substance that is subjected to fermentation is denoted "dairy product precursor" herein. The dairy product precursor may be made from fresh milk and/or milk powder, and may include further ingredients. As used herein, "milk" includes all variants thereof that can be used for producing a fermented diary product, such as whole milk, buttermilk, skim milk, fat-free milk, low fat milk, full fat milk, lactose-reduced milk, concentrated milk, etc. The dairy product precursor may differ depending on the fermented dairy product to be prepared. The fermented dairy product is the final product that is dispensed into packages and distributed for consumption by consumers. Examples of fermented dairy products include, but are not limited to, yoghurt, drinking yoghurt, concentrated yoghurt, sour cream, viili, kefir, ymer, skyr, cultured milk, cultured buttermilk, quark, koumiss, acidophilus milk, and any equivalent product.

As used herein, "intermediate product" is used as a common term for intermediate substances formed during the transition from the dairy product precursor into the fermented dairy product. Thus, the intermediate product may change in composition, texture, physical state, etc, during the process of manufacturing the fermented dairy product from the dairy product precursor.

As used herein, "bacterial culture" refers to any microbacterial culture that may be used to produce, by metabolization of one or more carbohydrates, a fermented dairy product from a dairy product precursor. The bacterial culture may include lactic acid bacteria (also known as LAB or lactobacillus) which produce lactic acid as their major metabolic end product of carbohydrate fermentation. For example, a bacterial culture for production of yoghurt conventionally contains *Streptococcus salivarius* ssp. *thermophilus* and *Lactobacillus delbrueckii* ssp. *bulgaricus* (also known as *Lactobacillus bulgaricus*)*.* It is also conceivable that the fermented dairy product is produced by a bacterial culture in combination with a yeast or another fungus.

Embodiments of the invention relate to techniques for preparation of fermented dairy products for ambient distribution and storage. Embodiments of the invention are provided to ensure that the fermented dairy product, when packaged for ambient distribution and storage, contains live bacteria, preferably the same type of bacteria that provide the fermentation of the dairy product precursor. Embodiments of the invention are also provided to control the microbial activity of the live bacteria, as well as any activity of unwanted microorganisms, in the fermented dairy product when packaged and exposed to ambient temperatures. As used herein, "ambient temperatures" refer to temperatures above about 10°C.

Embodiments of the invention are based on the insight that it is possible to provide a fermented dairy product that contains live bacteria by adding live bacteria subsequent to a final treatment for destruction of microorganisms, either as a starter culture for initiating the fermentation of the dairy product precursor or subsequent to the fermentation. Embodiments of the invention are also based on the insight that the microbial activity of the live bacteria in a fermented dairy product that comprises lactose may be controlled by providing the live bacteria as a specific or dedicated culture which is free of bacteria that consume lactose and which comprises lactic acid bacteria that consume at least one type of sugar other than lactose. As used herein, the sugar that is consumed by bacteria refers to the starting material for the metabolism by the bacteria, not the carbohydrate that is actually metabolized by the bacteria. Thus, "bacteria that consume lactose" comprise not only bacteria that metabolize lactose, but also bacteria that convert lactose into another sugar (e.g. glucose) and then metabolize this sugar. The microbial activity of such a dedicated culture is thus not driven by the availability of lactose but rather the availability of the specific type of sugar or sugars that is/are consumed by the lactic acid bacteria ("consumable sugar"). By controlling the availability of the consumable sugar, it is possible to obtain a controlled microbial activity in the fermented dairy product when packaged and exposed to ambient temperatures. Embodiments of the invention are preferably capable of producing packages containing a fermented dairy product with a shelf-life of at least 1 month, and even at least 2 months, at ambient temperatures in the range of 15-40°C.

Although embodiments of the invention provide distinct and apparent advantages for preparation of fermented dairy products that comprise lactose, they are equally applicable to preparation of fermented dairy products that are free or substantially free of lactose.

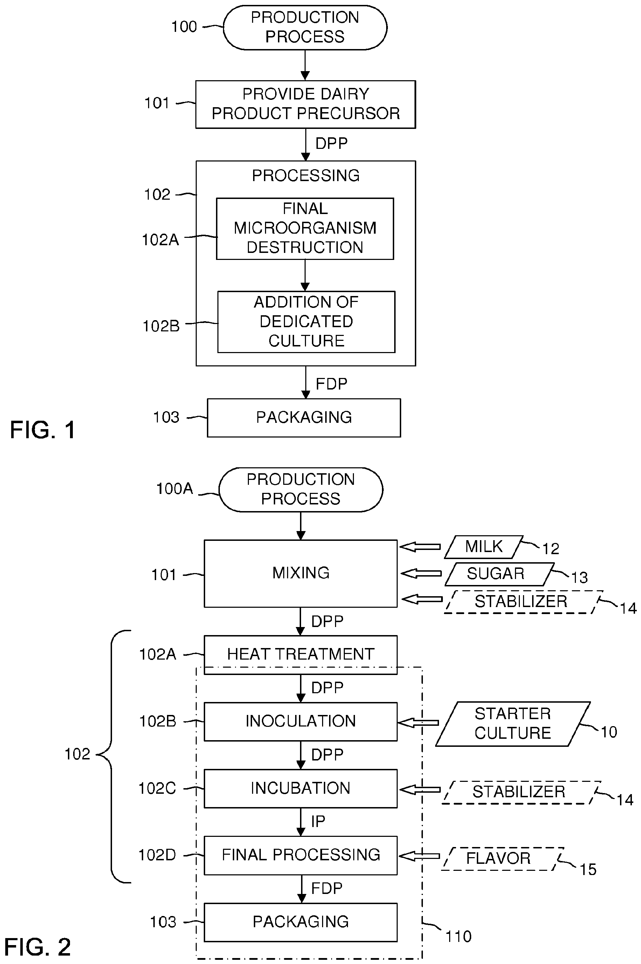

In the following, production processes that embody the foregoing insights will be further described and exemplified with reference to FIGS 1-3.

FIG. 1 is a flow chart of a production process 100 according to an embodiment of the invention. The process 100 comprises a step 101 of providing a dairy product precursor, denoted DPP in the following, as starting material for a subsequent fermentation. The DPP is also known as "milk base" in the art. Step 101 may be implemented in different ways. In one implementation, the DPP may be a premade substance, which is merely input as is to the manufacturing process 100. In another implementation, step 101 involves one or more processing sub-steps for preparing the DPP, e.g. by combining different ingredients. Such ingredients may comprise one or more milk-containing substances, as well as one or more of sugar, stabilizer, flavor and color, as is well-known in the art. In step 102, the DPP is processed into the fermented dairy product, denoted FDP in the following. In step 103, the FDP is dispensed, continuously or intermittently, into packages suitable for ambient distribution. Step 103 may be implemented in accordance with any conventional practice to produce a sealed receptacle, container or enclosure holding a quantity of the FDP to be stored at ambient temperature.

The processing 102 may involve a plurality of process steps, which transform the DPP into the FDP. As noted above, these process steps may be seen to generate an intermediate product from the DPP and transform the intermediate product into the FDP. It is realized that at least one of the process steps results in fermentation of the DPP or the intermediate product. FIG. 1 illustrates two further process steps 102A, 102B which are executed in sequence, although one or more intervening steps may be present. In step 102A, a final treatment for destruction of microorganisms is performed on either the DPP as supplied by step 101 or on the intermediate product. Step 102A may be performed before or after the fermentation to kill patogens and spoilage microorganisms, so as to prevent them from growing in the FDP when exposed to ambient temperatures. As used herein, "final treatment" or "final destruction treatment" refers to the last process step for destruction of microorganisms during the production process 100. Thus, subsequent to the final destruction treatment, the production process 100 is void of further treatment for destruction of microorganisms in the dairy product precursor, the intermediate product and the fermented dairy product. Generally, dairy products are currently required by law to have undergone a processing by one or more thermal methods to ensure a product free from pathogens. Such thermal methods are commonly referred to as "heat treatment" and involves heating the DPP or the intermediate product to a predefined temperature for a predefined time period, also known as "holding time". In one non-limiting example, the heat treatment may involve a heating to at least 72°C for at least 20 seconds. However, step 102A may also, or alternatively, comprise one or more non-thermal processes for destruction of microorganisms. Such non-thermal processes may include one or more of microfiltration, bactofugation and ultraviolet (UV) treatment, which are all techniques well-known to the person skilled in the art.

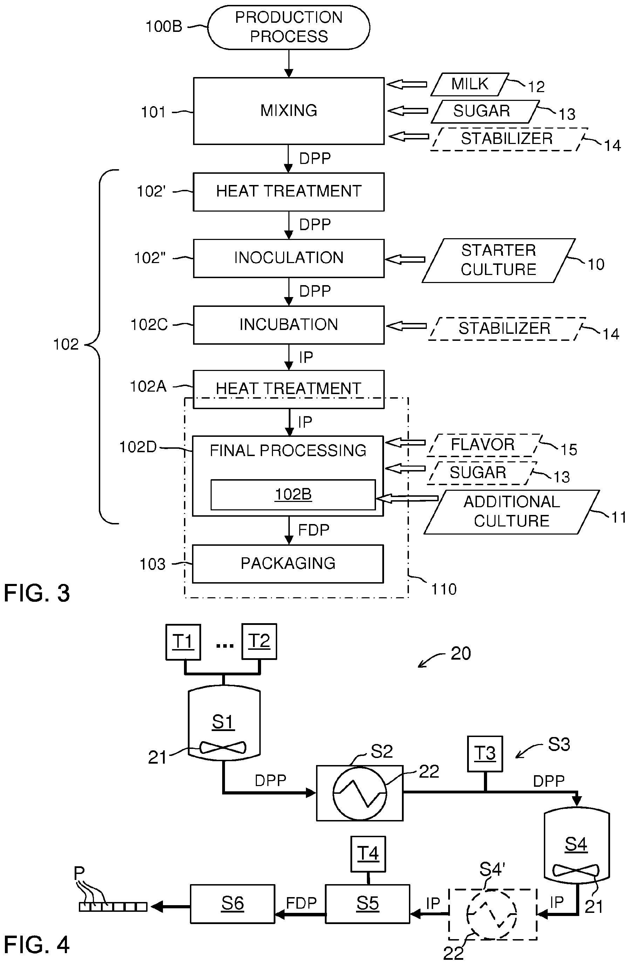

In step 102B, the dedicated culture is added to the DPP or the intermediate product. Depending on implementation, the dedicated culture may be provided as a starter culture for the fermentation or as an additional culture subsequent to the fermentation. These implementation concepts will be separately exemplified below with reference to FIG. 2 and FIG. 3, respectively. It should be realized that, irrespective of implementation concept, the addition of the dedicated culture enables the FDP to contain a predefined amount of live lactic acid bacteria that consume at least one type of sugar other than lactose while being essentially free of live bacteria that consume lactose. Presence of live bacteria that consume lactose in the FDP would potentially cause an uncontrolled growth of such bacteria when the FDP is exposed to ambient temperatures, if the FDP contains lactose. On the other hand, the growth of the lactic acid bacteria that are added by the dedicated culture will be limited by the availability of the specific sugar(s) consumed by these lactic acid bacteria, rather than temperature. Provided that the available amount of such consumable sugar in the FDP is properly restricted, uncontrolled growth of the lactic acid bacteria in the FDP may be prevented even at ambient temperatures.

The lactic acid bacteria in the dedicated culture may be selected to consume any suitable monosaccharide and/or any suitable disaccharide other than lactose. In one embodiment, the consumable sugar includes one or more of glucose, fructose, galactose, sucrose, and maltose. The dedicated culture may be selected among any current or future bacterial culture with appropriate properties. For example, the dedicated culture may be a bacterial culture that is commercially available from the company Chr. Hansen Holding A/S under the brand name ACIDIFIX®.

If the DPP contains no or only small amounts of the sugar(s) consumed by the lactic acid bacteria in the dedicated culture, the production process 100 may comprise a step of providing a predetermined amount of the consumable sugar. The consumable sugar may be provided by addition of the consumable sugar and/or by chemical decomposition of another sugar into the consumable sugar, e.g. by hydrolysis. In one embodiment, the predetermined amount of the consumable sugar may be at least partly provided during step 101. In one embodiment, the predetermined amount of the consumable sugar is set in relation to the amount of the lactic acid bacteria in the dedicated culture, e.g. to tailor the available amount of consumable sugar for the fermentation. In one embodiment, the predetermined amount of the consumable sugar is set so as to be completely consumed at a predefined time point, taking into account any amount of the consumable sugar that may be naturally present in milk. Thereby, it is possible to tailor the termination of the fermentation provided by the lactic acid bacteria of the dedicated culture. For example, the predefined time point may be set before the packaging step 103, to ensure that the FDP contains live bacteria when filled into the packages while also ensuring that these live bacteria do not continue to multiply or continue to ferment the FDP in the packages. In another example, the predefined time point may be a given time after the packaging step 103, to enable a controlled continued fermentation of the FDP within the respective package.

FIG. 2 illustrates a production process 100A which corresponds to the production process 100 of FIG. 1. The production process 100A comprises steps 101-103 and is an example embodiment of a first implementation concept for process steps 102A, 102B. According to the first implementation concept, step 102A is performed before fermentation, and step 102B adds the dedicated culture as a starter culture for fermentation. The illustrated example embodiment is e.g. useful for production of yoghurt. In the specific example shown in FIG. 2, step 101 involves a mixing of ingredients for the DPP. As shown, the ingredients may include one or more milk substances 12 and one or more sugars 13, and possibly one or more stabilizers 14. A stabilizer 14 may e.g. be added in step 101 for production of an FDP with high viscosity. The processing 102 includes a sequence of process steps, starting with the above-mentioned final treatment 102A for destruction of microorganisms. In the illustrated example, step 102A involves a conventional heat treatment of the DPP and subsequent cooling to a fermentation temperature. The heat treatment may serve the additional purpose of denaturating milk proteins so as to increase the water-binding capacity of the DPP and hence the firmness of coagulum generated by the subsequent fermentation. Step 102A is then followed by inoculation 102B, in which a starter culture 10 is added to the DPP. In the first implementation concept, as noted above, the starter culture 10 consists of the dedicated culture. The inoculation 102B is followed by incubation 102C, in which the DPP with the added starter culture 10 may be held at suitable fermentation temperature for a predefined time period, so as to cause the lactic acid bacteria in the starter culture to metabolize the consumable sugar and thereby provide an intermediate product, IP, with a desired fermentation. In a non-limiting example, the incubation 102C may have a duration of 4-12 hours. The incubation 102C may also involve one or more periods of agitation, e.g. for mixing the starter culture with the DPP and/or to break the coagulum that is formed during the fermentation. As indicated in FIG. 2, the incubation 102C may also involve adding one or more stabilizers 14. For example, a stabilizer 14 may be added in step 102C instead of step 101 for production of an FDP with low viscosity. Of course, it is conceivable that stabilizers 14 are added in both steps 101, 102C. The incubation 102C is followed by a final processing 102D that produces the FDP from the IP generated by the incubation 102C. As shown, the final processing 102D may involve adding flavor 15 to the IP. Generally, the final processing 102D may involve any type of desired or required processing of the IP, such as addition of one or more ingredients (e.g. flavor, sugar, stabilizer, color), mixing, agitation, cooling, concentration, homogenization, etc. In step 103, the FDP is then dispensed into packages for ambient distribution.

In the production process 100A, fermentation may be controlled by adjusting the availability of the consumable sugar, in the same way as described for the process 100 of FIG. 1. For example, at least part of a metered amount of the consumable sugar may be added with sugar 13 in step 101, possibly with further consumable sugar being added during step 102C and/or step 102D. Depending on the available amount of the consumable sugar, the fermentation may be confined to the incubation 102C or proceed during at least part of the final processing 102D, or even continue for a predefined time period within the packages produced by step 103.

The first implementation concept enables the FDP to contain, at least at the time of packaging, a live bacterial culture that originates from the dedicated culture that was added at the inoculation 102B. The first implementation concept also enables the FDP to be free of bacteria that consumes lactose. Further, by performing the final destruction treatment 102A before the fermentation, the impact of the final destruction treatment on the FDP is minimized. For example, the well-known drawbacks of performing a heat treatment after fermentation are mitigated. Thus, the first implementation concept has the potential of reducing the perceived difference in taste and/or texture between packaged FDP for chilled and ambient distribution.

To ensure the quality of the FDP when packaged and exposed to ambient temperatures, it is desirable to limit the risk for contamination of DPP, IP or FDP by unwanted microorganisms subsequent to the final destruction treatment 102A. Preferably, as indicated by a rectangle 110 in FIG. 2, all steps subsequent to the final destruction treatment 102A are performed at aseptic conditions and/or high hygiene conditions, in accordance with well-established procedures, to prevent such contamination. As used herein, "high hygiene conditions" imply that the relevant production equipment has been sterilized before production by exposure to temperatures of at least 95°C, e.g. by hot water and/or steam sterilization.

FIG. 3 illustrates a production process 100B which corresponds to the production process 100 of FIG. 1. The production process 100B comprises steps 101-103 and is an example embodiment of a second implementation concept for steps 102A, 102B. According to the second implementation concept, step 102A is performed after or during fermentation, and step 102B adds the dedicated culture to ensure that the FDP contains live bacteria. The illustrated embodiment is e.g. useful for production of yoghurt. For brevity of presentation, steps that are identical to steps in FIG. 2 will not be reiterated in detail. This applies to step 101 which provides the DPP. The processing 102 includes a sequence of process steps, starting with an initial destruction treatment 102' of the DPP for destruction of microorganisms, which may involve a conventional heat treatment and a subsequent cooling. The initial destruction treatment 102' is then followed by inoculation 102", in which a starter culture 10 is added to the DPP. In the second implementation concept, the starter culture 10 need not be the dedicated culture but could comprise any type of bacterial culture. The inoculation 102" is followed by incubation 102C, e.g. as described with reference to FIG. 2. After the incubation 102C, the resulting intermediate product, IP, is subjected to the final destruction treatment 102A, which may be performed as described above, thereby destructing all microorganisms in the IP including all bacteria originating from the starter culture 10. The final destruction treatment 102A is followed by the final processing 102D that produces the FDP from the IP. The final processing 102D may comprise any type of desired or required processing of the IP, e.g. as described with reference to FIG. 2. As indicated in FIG. 3, the final processing 102D also comprises the step 102B of adding the dedicated culture, represented by additional culture 11 in FIG. 3. In step 103, the FDP is then dispensed into packages for ambient distribution. Preferably, as indicated by the rectangle 110 in FIG. 3, all steps subsequent to the final destruction treatment 102A are performed at aseptic conditions and/or high hygiene conditions.

The second implementation concept enables the FDP to contain, at least at the time of packaging, a live bacterial culture that originates from the dedicated culture added during the final processing 102D. The second implementation concept also enables the FDP to be free of bacteria that consume lactose.

The survival and potential growth of the live bacterial culture in the FDP may be controlled, as described above, by adjusting the availability of consumable sugar for the dedicated culture. For example, sugar 13 added in step 101 may comprise the above-mentioned predetermined amount of consumable sugar. It should be understood that the sugar 13 added in step 101 may also comprise sugar to be consumed by the starter culture 10, which may differ from the additional culture 11. Alternatively or additionally, sugar(s) for consumption by the dedicated culture may be added with sugar 13 (FIG. 3) in the final processing 102D and/or in step 102C.

FIG. 4 illustrates an arrangement or production line 20 for producing packages P containing FDP for ambient distribution. The arrangement 20 comprises a sequence of stations that are configured to perform any of the production processes 100A, 100B described in the foregoing. The individual stations may be configured for batch processing or continuous flow processing, as is known in the art. In fact, each station may be of conventional construction for its intended purpose. In the illustrated example, the arrangement 20 comprises a preparatory station S1 for preparation of the DPP in accordance with step 101. Station S1 is connected to or comprises one or more tanks (two shown: T1, T2) holding either the DPP or ingredients for the DPP, e.g. milk substance 12 and sugar 13. In the illustrated example, station S1 comprises a stirring element 21, which may be operated for agitating the DPP and/or for mixing the ingredients. Station S2 comprises a heater/cooler 22 and is configured to perform either the final destruction treatment 102A of process 100A or the initial destruction treatment 102' of process 100B. Station S3 is configured to perform the inoculation (102B in process 100A; 102" in process 102B) and comprises a tank T3 containing the starter culture. Station S4 is configured to perform the incubation 102C and comprises a stirring element 21, which may be operated for agitating the coagulum that is formed by the fermentation in station S4 and/or for admixing one or more ingredients that may be added to the coagulum. Station S4' comprises a heater/cooler 22 and is configured to perform the final destruction treatment 102A of process 100B. Station S4' is omitted when the arrangement 20 is configured to perform process 100A (FIG. 2). Station S5 is configured to perform the final processing 102D. In the illustrated example, station S5 is connected to or comprises one or more tanks (one shown: T4) holding substances to be added during the final processing 102D. When the arrangement 20 is configured to perform the final processing 102D of process 100B, tank T4 may contain the additional culture 11. Station S6 is a packaging station configured to perform the dispensing and packaging in accordance with step 103, to produce the packages P for ambient distribution.

## Claims

1. A method of producing packages (P) containing a fermented dairy product (FDP) for ambient distribution, said method comprising:
providing (101) a dairy product precursor (DPP);
processing (102) the dairy product precursor (DPP) to generate the fermented dairy product (FDP) via an intermediate product (IP); and
dispensing (103) the fermented dairy product (FDP) into the packages (P) for ambient distribution;
wherein said processing (102) comprises:
performing, on the dairy product precursor (DPP) or the intermediate product (IP), a final treatment (102A) for destruction of microorganisms, and
adding (102B) to the dairy product precursor (DPP) or the intermediate product (IP), subsequent to the final treatment (102A), a dedicated culture which is free of bacteria that consume lactose and which comprises lactic acid bacteria that consume at least one type of sugar other than lactose, so that the fermented dairy product (FDP) in the packages (P) comprises a live bacterial culture originating from the dedicated culture.

2. The method of claim 1, wherein said processing (102), subsequent to the final treatment (102A), and said dispensing (103) are performed at aseptic conditions and/or high hygiene conditions.

3. The method of any preceding claim, wherein said processing (102) comprises: performing the final treatment (102A) on the dairy product precursor (DPP) for destruction of microorganisms therein.

4. The method of claim 3, wherein said adding (102B) comprises: adding (102B) to the dairy product precursor (DPP), subsequent to the final treatment (102A), a starter culture (10) that consists of the dedicated culture; and
wherein said processing (102) further comprises:
incubating (102C) the dairy product precursor (DPP) with the added starter culture (10) to produce the intermediate product (IP), and
producing (102D) the fermented dairy product (FDP) from the intermediate product (IP) so that the fermented dairy product (FDP) comprises the live bacterial culture originating from the dedicated culture.

5. The method of any one of claims 1-2, wherein said processing (102) comprises:
adding (102") a starter culture (10) to the dairy product precursor (DPP),
incubating (102C) the dairy product precursor (DPP) with the added starter culture (10) to produce the intermediate product (IP), and
performing the final treatment (102A) on the intermediate product (IP) for destruction of microorganisms therein.

6. The method of claim 5, wherein said adding (102B) comprises: adding (102B) to the intermediate product (IP), subsequent to the final treatment (102A), an additional culture (11) that consists of the dedicated culture.

7. The method of any preceding claim, further comprising providing a predetermined amount of a sugar (13) to be consumed by the dedicated culture.

8. The method of claim 7, wherein the predetermined amount of the sugar (13) is at least partly provided during the step of providing (101) the dairy product precursor (DPP).

9. The method of claim 7 or 8, wherein the predetermined amount of the sugar (13) is set in relation to the amount of the lactic acid bacteria in the dedicated culture.

10. The method of any one of claims 7-9, wherein the predetermined amount of the sugar (13) is set so as to be completely consumed at a predefined time point.

11. The method of claim 10, wherein the predefined time point is before said dispensing (103) or a given time after said dispensing (103).

12. The method of any preceding claim, wherein the packages (P) are produced to have a shelf-life of at least 1 month, or at least 2 months, at ambient temperatures in a range of 15-40°C.

13. The method of any preceding claim, wherein the fermented dairy product (FDP) in the packages (P) comprises lactose.

14. A package produced by the method as claimed in any one of the preceding claims.

15. An arrangement for producing packages (P) containing a fermented dairy product (FDP) for ambient distribution, said arrangement comprising:
a preparatory station (S1) configured to provide a dairy product precursor (DPP),
a sequence of processing stations (S2-S5) configured to process the dairy product precursor (DPP) into the fermented dairy product (FDP) via an intermediate product (IP), and
a packaging station (S6) configured to receive the fermented dairy product (FDP) from the sequence of processing stations (S2-S5) and dispense the fermented dairy product (FDP) into the packages (P) for ambient distribution,
wherein the sequence of processing stations (S2-S5) comprises:
a first station (S2; S4') which is configured to perform, on the dairy product precursor (DPP) or the intermediate product (IP), a final treatment for destruction of microorganisms, and
a second station (S3; S5) which is arranged downstream of the first station (S2; S4') and configured to add, to the dairy product precursor (DPP) or the intermediate product (IP), a dedicated culture which is free of bacteria that consume lactose and which comprises lactic acid bacteria that consume at least one type of sugar other than lactose, so that the fermented dairy product (FDP) comprises a live bacterial culture originating from the dedicated culture.
